# EUROPEAN PATENT SPECIFICATION

(11) **EP 4 186 492 B1**
(45) Date of publication and mention of the grant of the patent: **21.01.2026**
(21) Application number: 21846100.2
(22) Date of filing: 23.07.2021
(51) Int. Cl.: A61K 9/08, A61K 47/12, A61K 47/02, A61K 47/22, A61K 47/26, A61K 47/34, A61K 9/00, A61K 38/17, A61K 39/395, A61K 47/18

(54) **OPHTHALMIC LIQUID COMPOSITION**
FLÜSSIGE OPHTHALMISCHE ZUSAMMENSETZUNG
COMPOSITION LIQUIDE OPHTALMIQUE

(30) Priority: 24.07.2020 KR 20200092433
(43) Date of publication of application: 31.05.2023
(73) Proprietor: Pangen Biotech Inc., Gyeonggi-do 16675 (KR)
(72) Inventor: YOON, Jaeseung, Yongin-si Gyeonggi-do 16871 (KR); PARK, Jeong Soo, Yongin-si Gyeonggi-do 16923 (KR); LEE, Seunghee, Seoul 06262 (KR); KIM, Hyunjoo, Hwaseong-si Gyeonggi-do 18396 (KR)
(74) Representative: Algemeen Octrooi- en Merkenbureau B.V.
(86) International application number: PCT/KR2021/009593
(87) International publication number: WO 2022/019721

(56) References cited:
- WO-A1-2018/094316
- KR-A- 20150 033 620
- KR-A- 20170 138 387
- KR-A- 20200 029 374
- KR-A- 20200 084 897
- KR-A- 20200 084 897
- US-A1- 2017 080 086

## Description

### TECHNICAL FIELD

The present invention relates to an ophthalmic liquid composition comprising a therapeutically effective amount of aflibercept; a buffer containing sodium acetate or histidine; a sugar including sucrose, trehalose or a combination thereof; and a surfactant.

### BACKGROUND ART

Aflibercept is a clear, colorless to pale yellow solution for injection and known as a therapeutic agent for diseases caused by abnormal angiogenesis by a vascular endothelial growth factor (VEGF), one of the factors related to angiogenesis and increased vascular permeability, for example, wet age-related macular degeneration, diabetic macular edema, and macular edema in retinal vein occlusion.

Like other therapeutic protein drugs, aflibercept may also undergo stress under suboptimal conditions, for example, physical and chemical deterioration during storage and transportation. In particular, the oxidation, deamidation, isomerization or polymerization of a protein may occur depending on the concentration of the protein, type and concentration of the stabilizer, pH, temperature, salt concentration, external impact and contact with air/light, etc., which may lead to the formation of aggregates, fragments and isomers of the protein drug to reduce the activities of the drug. In addition, it is the nature of an ophthalmic liquid composition that the formation of particulates due to aggregation in a protein drug may cause side effects. Thus, the development of a formulation capable of stabilizing same is most important.

Therefore, the inventors of the present invention have made diligent efforts to develop a formulation with improved stability against various stresses that may occur during pharmaceutical processing, storage and transportation of the ophthalmic liquid composition comprising aflibercept and have completed the present invention by confirming that the ophthalmic liquid composition of the present invention comprising an isotonic agent (e.g., sodium acetate, histidine, sodium chloride or sorbitol) in the buffer at a certain concentration showed an excellent effect of imparting stability against stress upon agitation, repeated freezing and thawing, ultraviolet (UV) exposure, and storage at a high temperature.

International Patent Publication WO 2007/149334 discloses an ophthalmic, lyophilized formulation comprising aflibercept, an organic co-solvent such as polysorbate, an ionic tonicity agent selected from sodium chloride and potassium chloride; a sodium phosphate buffer; and a stabilizer such as sucrose, wherein the formulation has a pH of 5.8 to 7.0. However, even in such a formulation, the stability of aflibercept is significantly reduced under stressed conditions such as a high temperature (e.g., 40 °C). Therefore, it is necessary to develop an aflibercept ophthalmic liquid composition that provides improved stability even under various stressed conditions comprising such a high temperature. International Patent Publication WO 2018/094316 A1 presents a composition comprising aflibercept that are suitable for a method of treatment of an eye disorder or disease by intravitreal or topical administration. Korean Patent Publication KR 2020-0084897 A presents a composition for stabilizing a protein containing a buffer; stabilizer; one or more selected from the group consisting of trehalose, sucrose, and mannitol, and a method for preparing a stabilized liquid composition by using the composition for stabilization.

Accordingly, the inventors of the present invention have made diligent efforts to develop a formulation of an ophthalmic liquid composition comprising aflibercept with improved stability against various stressed conditions and have completed the present invention by confirming that the ophthalmic liquid composition of the present invention comprising a buffer containing sodium acetate or histidine; a sugar including sucrose, trehalose or a combination thereof; and a surfactant showed improved stability even under stressed conditions for a long period of time.

### SUMMARY OF THE INVENTION

### TECHNICAL PROBLEM

An object of the present invention is to solve the above-described problems and other problems related thereto.

One exemplary object of the present invention is to provide an ophthalmic liquid composition comprising a therapeutically effective amount of aflibercept; a buffer containing sodium acetate or histidine; a sugar including sucrose, trehalose or a combination thereof; and a surfactant.

Another exemplary object of the present invention is to provide a composition for the stabilization of aflibercept, comprising a buffer containing sodium acetate or histidine; a sugar including sucrose, trehalose or a combination thereof; and a surfactant.

Another exemplary object of the present invention is to provide a container comprising the ophthalmic liquid composition.

Another exemplary object of the present invention is to provide a method for preventing or treating ophthalmic diseases, comprising administering the ophthalmic liquid composition to a subject.

Another exemplary object of the present invention is to provide use of the ophthalmic liquid composition for preventing or treating ophthalmic diseases.

Another exemplary object of the present invention is to provide use of the ophthalmic liquid composition for the preparation of a medicament for preventing or treating ophthalmic diseases.

The technical problem to be achieved according to the technical idea of the invention disclosed in the present specification is not limited to the above-described problems, and another problem not mentioned above may be clearly understood by those skilled in the art from the description below.

### TECHNICAL SOLUTION

A detailed description of the present invention is as follows. The description and embodiment described in the specification of the present invention may also be applied to other descriptions and embodiments. That is, all combinations of various elements described in the specification of the present invention fall within the scope of the present invention. In addition, the scope of the present invention is not limited by the specific descriptions described below.

One aspect of the present invention for achieving the above-described object is to provide an ophthalmic liquid composition comprising a therapeutically effective amount of aflibercept; a buffer containing sodium acetate or histidine; a sugar including sucrose, trehalose or a combination thereof; and a surfactant.

As described above, the ophthalmic liquid composition of the present invention shows an excellent effect of imparting stability against stress upon agitation, repeated freezing and thawing, ultraviolet (UV) exposure and storage at a high temperature, thereby minimizing stress under suboptimal conditions, for example, deterioration that may physically and chemically occur during storage and transportation, and stably maintaining the activities of the drug, as compared to formulations comprising aflibercept conventionally known in the art.

In the present invention, the term "aflibercept" is the name of an active ingredient contained in ophthalmic injections developed by Regeneron Pharmaceuticals and sold under the brand name EYLEA^{®} as a therapeutic agent for macular degeneration and refers to a fusion protein comprising the extracellular domain of VEGFR1 or VEGFR2 fused to the Fc region of human IgG1.

In the present invention, the aflibercept may be contained at a concentration of 10 to 100 mg/mL, particularly 10 to 40 mg/mL, and more particularly 40 mg/mL. Also, in the present invention, the term "aflibercept" comprises not only aflibercept but also biosimilars thereof.

In the present invention, the term "biosimilar" refers to a generic drug for a biological drug that may be produced by a third party other than the original patent holder because the patent has expired, it is also referred to as a biogeneric. That is, if a drug has the same amino acid sequence information as a patented biopharmaceutical and is not manufactured in the same process but produces almost the same biological effect as a result of a blood test through clinical trials, the drug is certified to be equivalent to the patented biopharmaceutical and recognized as a biosimilar thereof.

In the present invention, the term "buffer" refers to a solution comprising a weak acid and its conjugate base or a weak base and its conjugate acid and capable of withstanding pH changes caused by external factors. In the present invention, the buffer may comprise sodium acetate or histidine and have a pH of 5.3 to 6.2, more particularly, a pH of 5.5 to 5.8.

The sodium acetate or histidine may have a concentration of 5 to 20 mM, preferably 5 to 10 mM.

In the present invention, the sugar comprises sucrose, trehalose or a combination thereof, and the sugar may be present at a concentration of 5 to 20% (w/v), particularly 8 to 10% (w/v).

In the present invention, the surfactant may be polysorbate or poloxamer and, particularly, 0.01 to 0.03% (w/v) of polysorbate 20, polysorbate 80 or poloxamer 188.

In addition, the ophthalmic liquid composition of the present invention may further comprise an isotonic agent such as sodium chloride and sorbitol, wherein the sodium chloride may be present at a concentration of 10 to 50 mM, more particularly, 20 to 30 mM, and the sorbitol may be present at a concentration of 1 to 10% (w/v).

Also, a salt such as sodium chloride may not be contained in the composition for stability thereof.

In addition, as confirmed in one embodiment, after conducting SE-HPLC, cIEF, RP-HPLC, deamidation, binding assay, etc. under accelerated (e.g., at 25°C) and stressed (e.g., at 40°C) conditions, it was confirmed that the formulation having a high pH or comprising a salt showed an increase in the formation of aggregates and isomers with a low pI, and the formulation having a very low pH showed an increase in the formation of fragments and oxidants; and the formulation having a pH of 5.5 to 5.8 and containing no salt such as NaCl was the most stable formulation.

The ophthalmic liquid composition of the present invention is, for example, an injectable composition, characterized by having a formulation suitable for intravitreal injection and, in particular, may be applied to a pre-filled syringe suitable for intravitreal injection.

The ophthalmic liquid composition may be used for medicinal purposes for various diseases that may be improved/treated by treatment with aflibercept, without particular limitation, and preferably used for various ophthalmic diseases (e.g., retinal vein occlusion, diabetic macular edema, choroidal neovascularization and wet age-related macular degeneration). In addition, the ophthalmic liquid composition may further comprise a pharmaceutically acceptable carrier, an excipient, or a diluent conventionally used in the art, and the present invention is not particularly limited thereto.

A suitable dosage of the ophthalmic liquid composition of the present invention may vary depending on factors (e.g., a preparation method, an administration method, patient's age, weight, sex and morbid condition, food, an administration time, an administration route, an excretion rate and a response sensitivity). The doctor can easily determine and prescribe an effective dosage for treatment or prevention.

Another aspect of the present invention for achieving the above-described object is to provide a composition for the stabilization of aflibercept comprising a buffer containing sodium acetate or histidine; a sugar including sucrose, trehalose or a combination thereof; and a surfactant. The definition, type and concentration of the term "aflibercept," "buffer," "sugar" and "surfactant" are as described above.

The composition of the present invention is characterized by showing an excellent stabilizing effect under stressed conditions (e.g., agitation, repeated freezing and thawing, ultraviolet (UV) exposure and a high temperature) depending on a storage temperature. In the present invention, the term "stability" refers to maintaining physical and/or chemical stability against various stresses that may occur when the protein contained in a drug is not under optimal conditions for pharmaceutical processing, storage and transportation, and retaining biological stability to maintain activities as an ophthalmic liquid composition. The stability of a protein or a drug can be measured by appropriately employing various analytical techniques by those skilled in the art and adopting particular temperatures, stressed conditions, periods and intervals of time.

Specifically, in order to confirm the stability of the composition of the present invention under stressed conditions (e.g., agitation, repeated freezing and thawing, and UV conditions), visual inspection, size exclusion chromatography (SE-HPLC) and FlowCAM analysis were performed to determine whether or not aggregates were formed, and stability was evaluated during storage at various temperatures (e.g., -70 °C, 5 °C, 25 °C and 40 °C). As a result, it was confirmed that the composition of the present invention showed excellent stability under stressed conditions (e.g., agitation, repeated freezing and thawing, ultraviolet (UV) exposure and high temperature). In addition, the formation of oxidants through reversed-phase chromatography (RP-HPLC), protein degradation through deamidation analysis, and binding activities of aflibercept through binding assays were verified. As a result, it was confirmed that the composition of the present invention showed excellent stability.

Another aspect of the present invention for achieving the above-described object is to provide a container comprising the ophthalmic liquid composition or the composition for stabilization (e.g., a vial, an ampoule and a pre-filled syringe). Such a container may be packaged and provided in a sealed form.

As used herein, the term "prefilled syringe" refers to a prefilled syringe in which a drug is directly filled, and is a syringe in a ready-to-use form in which the composition of the present invention is pre-filled and stored. The pre-filled syringe of the present invention may be a plastic syringe or a glass syringe, filled with the composition of the present invention, and applied by intravitreal injection by attaching a sterile needle (e.g., injection needle) to the inlet of the pre-filled syringe.

Another aspect of the present invention for achieving the above-described object is to provide a kit comprising a container containing the ophthalmic liquid composition or the composition for stabilization and an instruction manual to be provided to a user. Such a kit comprises presentation forms conventionally known in the art.

Another aspect of the present invention for achieving the above-described object is to provide a method for preventing or treating ophthalmic diseases, comprising administering the ophthalmic liquid composition to a subject.

As described above, the ophthalmic liquid composition provided in the specification of the present application may be used for medicinal purposes for various diseases that may be improved/treated by the treatment of aflibercept, without particular limitation, and preferably used to prevent or treat various ophthalmic diseases (e.g., retinal vein occlusion, diabetic macular edema, choroidal neovascularization and wet age-related macular degeneration). That is, the composition may be used to prevent or treat ophthalmic diseases.

The term "individual" of the present invention comprises, without limitation, mammals comprising mice, livestock and human beings that are likely to develop or have developed an ophthalmic disease.

A suitable dosage of the ophthalmic liquid composition of the present invention may vary depending on factors (e.g., a preparation method, an administration method, patient's age, weight, sex and morbid condition, food, an administration time, an administration route, an excretion rate and a response sensitivity). The doctor can easily determine and prescribe an effective dosage for treatment or prevention.

Another aspect of the present invention for achieving the above-described object is to provide use of the ophthalmic liquid composition for preventing or treating ophthalmic diseases.

Another aspect of the present invention for achieving the above-described object is to provide use of the ophthalmic liquid composition for the preparation of a medicament for preventing or treating ophthalmic diseases.

### ADVANTAGEOUS EFFECTS

The ophthalmic liquid composition of the present invention variously adjusts the buffer composition, pH range, etc. to improve physicochemical and biological stability, reduce the formation of aggregates even under stressed conditions (e.g., agitation, repeated freezing and thawing, ultraviolet (UV) exposure and a high temperature) and maintain stability and activities even under stressed conditions (e.g., refrigerated storage conditions, accelerated conditions and a high temperature) and, thus, may be usefully applied to the treatment of various ophthalmic diseases.

### BRIEF DESCRIPTION OF THE DRAWINGS

FIG. 1 is the result of confirming an increase in the formation of related substances in the ophthalmic liquid composition of the present invention according to agitation.
FIG. 2 is the result of confirming an increase in the formation of related substances in the ophthalmic liquid composition of the present invention according to repeated freezing and thawing.
FIG. 3 is the result of confirming an increase in the formation of related substances in the ophthalmic liquid composition of the present invention according to ultraviolet (UV) exposure.
FIG. 4 is the result of confirming a change in the properties of the ophthalmic liquid composition of the present invention at -70 °C.
FIG. 5 is the result of confirming a change in the properties in the ophthalmic liquid composition of the present invention at 5 °C.
FIG. 6 is the result of confirming a change in the aggregates in the ophthalmic liquid composition of the present invention at -70 °C.
FIG. 7 is the result of confirming a change in the aggregates in the ophthalmic liquid composition of the present invention at 5 °C.
FIG. 8 is the result of confirming a change in the aggregates or fragments in the ophthalmic liquid composition of the present invention at 25 °C.
FIG. 9 is the result of confirming a change in the aggregates or fragments in the ophthalmic liquid composition of the present invention at 40 °C.

### DETAILED DESCRIPTION OF THE EMBODIMENTS

Hereinafter, the present invention will be described in more detail through the following examples. However, these examples are intended to illustrate the present invention by way of example, and the scope of the present invention is not limited only to these examples.

### Example 1: Preparation of an ophthalmic liquid composition according to the present invention

The ophthalmic liquid composition according to the present invention was prepared with the following ingredients and contents.

**[Table 1]**

| **Formulation** | **Buffer (10 mM)** | **pH** | **Sugar** | **Active ingredient (mg/mL)** | **Surfactant** |
|---|---|---|---|---|---|
| 1 | Sodium acetate | 5.3 | 10% Trehalose | Aflibercept 40 | 0.01% PS20 |
| 2 | Sodium acetate | 5.3 | 10% Sucrose | Aflibercept 40 | 0.01% PS20 |
| 3 | Sodium acetate | 5.5 | 10% Trehalose | Aflibercept 40 | 0.01% PS20 |
| 4 | Sodium acetate | 5.5 | 10% Sucrose | Aflibercept 40 | 0.01% PS20 |
| 5 | Sodium acetate | 5.8 | 8% Trehalose | Aflibercept 40 | 0.01% PS20 |
| | | | 25 mM NaCl | | |
| 6 | Sodium acetate | 5.8 | 8% Sucrose | Aflibercept 40 | 0.01% PS20 |
| | | | 25 mM NaCl | | |
| 7 | Histidine | 5.8 | 10% Trehalose | Aflibercept 40 | 0.01% PS20 |
| 8 | Histidine | 5.8 | 10% Sucrose | Aflibercept 40 | 0.01% PS20 |
| 9 | Histidine | 6.0 | 10% Trehalose | Aflibercept 40 | 0.01% PS20 |
| 10 | Histidine | 6.0 | 10% Sucrose | Aflibercept 40 | 0.01% PS20 |
| 11 | Histidine | 6.2 | 10% Trehalose | Aflibercept 40 | 0.01% PS20 |
| 12 | Histidine | 6.2 | 10% Sucrose | Aflibercept 40 | 0.01% PS20 |
| 13 | (Control) 40 mg/mL Aflibercept, 10 mM Sodium phosphate, 5% Sucrose, 40 mM NaCl, 0.03% PS20, pH 6.2 | | | | |

### Example 2: Evaluation of the stability of the ophthalmic liquid composition according to the present invention under accelerated/stressed conditions

The composition formulations prepared in Example 1 were put into a glass vial or a pre-filled syringe (PFS), and stability was evaluated under various conditions as follows.

The accelerated stability assessment comprises incubation of each of the composition formulations at storage temperatures of frozen (-70 °C), refrigerated (5 °C), accelerated (25 °C) and stressed (40 °C) conditions. In addition, the stability of each of the composition formulations was evaluated under other acute stress conditions related to processing and shipment of pharmaceuticals. These stressed conditions comprise agitation (e.g., shear stress), repeated freezing and thawing and ultraviolet (UV) exposure. Table 2 below summarizes each condition used for the accelerated/stressed stability evaluation.

**[Table 2]**

| **Accelerated/Stressed** | **Conditions** | **Point** |
|---|---|---|
| Temperature | -70 °C, 5 °C | 0 weeks, 4 weeks, 8 weeks, 3 months, 6 months and 12 months |
| | 25 °C, 40 °C | 0 weeks, 4 weeks, 8 weeks, 3 months and 6 months |
| Agitation | Vortexing at 1,000 rpm | 4 hours |
| Repeated freezing and thawing | -70 °C to room temperature | 5 consecutive cycles |
| Photosensitivity | UV exposure | UVA of 8 watt-hours/m² |

### Example 3. Evaluation of stability under acute stress conditions

### 3-1. Evaluation of stability under agitation conditions

All formulations prepared in Example 1 were exposed to constant agitation at 1,000 rpm at room temperature for 4 hours by using a bench-top vortexer. As control samples, the same set of formulations were incubated at room temperature for 4 hours without agitation, after which the properties and presence of visible particle formation were observed for each of the agitated and control formulations. To confirm the properties, visual inspection was performed by using a white light source (e.g., a 13 W fluorescent tube) to a black and white background, and digital photographs were obtained as results at all viewpoints. As a result, it was confirmed that there was no change in the properties of all of Formulations 1 to 12 of Example 1 due to agitation.

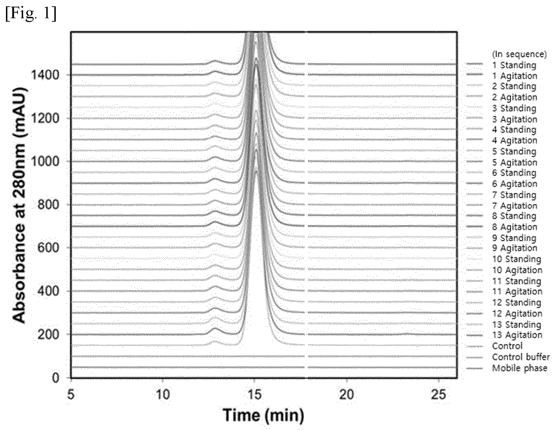

In addition, as a result of confirming changes in aggregates and fragments in SE-HPLC analysis performed by using a Hewlett Packard Agilent 1100 Series (conditions: column; TSK-GEL G3000SWXL, 7.8 X 30 (ID mm X L cm) (Tosoh, Cat. # 08541), mobile phase: 200 mM KPi, 250 mM KCl, pH 6.2, flow rate: 0.5 mL/min, and wavelength: 280 nm), it was confirmed that no increase in the formation of related substances was observed in all formulations of Example 1 under agitation conditions, as shown in Figure 1 and Table 3.

**[Table 3]**

| **Formulation** | **Conditions for treatment** | **Aggregate 2 (%)** | **Aggregate 1 (%)** | **Main peak (%)** |
|---|---|---|---|---|
| Control (-70 °C) | | 0.1 | 1.9 | 98.0 |
| 1 | Static | 0.1 | 1.8 | 98.1 |
| | Agitation | 0.1 | 1.8 | 98.1 |
| 2 | Static | 0.1 | 1.7 | 98.2 |
| | Agitation | 0.1 | 1.7 | 98.2 |
| 3 | Static | 0.1 | 2.1 | 97.8 |
| | Agitation | 0.1 | 2.1 | 97.8 |
| 4 | Static | 0.1 | 2.0 | 97.9 |
| | Agitation | 0.1 | 2.0 | 97.9 |
| 5 | Static | 0.1 | 2.4 | 97.5 |
| | Agitation | 0.1 | 2.4 | 97.5 |
| 6 | Static | 0.1 | 2.4 | 97.5 |
| | Agitation | 0.1 | 2.4 | 97.5 |
| 7 | Static | 0.1 | 2.3 | 97.6 |
| | Agitation | 0.1 | 2.3 | 97.6 |
| 8 | Static | 0.1 | 2.3 | 97.6 |
| | Agitation | 0.1 | 2.3 | 97.6 |
| 9 | Static | 0.1 | 2.3 | 97.6 |
| | Agitation | 0.1 | 2.3 | 97.6 |
| 10 | Static | 0.1 | 2.3 | 97.6 |
| | Agitation | 0.1 | 2.3 | 97.6 |
| 11 | Static | 0.1 | 2.5 | 97.4 |
| | Agitation | 0.1 | 2.5 | 97.4 |
| 12 | Static | 0.1 | 2.5 | 97.4 |
| | Agitation | 0.1 | 2.5 | 97.4 |
| 13 | Static | 0.1 | 3.2 | 96.7 |
| | Agitation | 0.1 | 3.2 | 96.7 |

### 3-2. Evaluation of stability under repeated freezing and thawing conditions

In order to evaluate stability against repeated freezing and thawing, all formulations of Example 1 were repeatedly frozen at -70 °C for 30 minutes or longer and thawed at room temperature for 30 minutes or longer 5 times in a row.

As a result of visually confirming complete freezing and thawing, it was confirmed that there was no change in the properties of all formulations due to repeated freezing and thawing.

In addition, SE-HPLC analysis (Hewlett Packard Agilent 1100 Series, column; TSK-GEL G3000SWXL, 7.8 X 30 (ID mm X L cm) (Tosoh, Cat. # 08541), mobile phase: 200 mM KPi, 250 mM KCl, pH 6.2, Flow rate: 0.5 mL/min, wavelength: 280 nm) was used to observe physical degradation of each formulation before and after freezing and thawing. To monitor the accuracy of the assay and provide a comparison between formulations, the chromatographic profile of each formulation was checked along with the injection of the control. The stability of each formulation was compared by confirming the formation of aggregates and fragments by peak percentage over time.

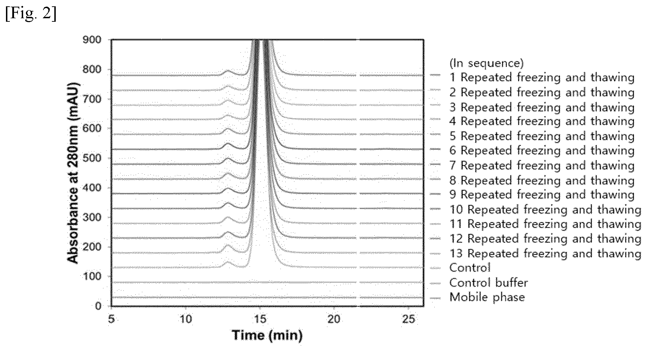

As a result of confirming changes in aggregates and fragments, it was confirmed that there was no increase in related substances of all formulations of Example 1 due to repeated freezing and thawing, as shown in FIG. 2.

In addition, FlowCAM analysis (Benchtop B3 Series, Fluid Imaging Technologies) was performed to monitor the degree of subvisible particles in each formulation before and after repeated freezing and thawing, and the stability of each formulation was evaluated by confirming changes in subvisible particles following repeated freezing and thawing. As a result, no significant increase in subvisible particles was observed in all formulations of Example 1. Through these analysis results, it was confirmed that the ophthalmic liquid composition of Example 1 was stable under repeated freezing and thawing conditions.

### 3-3. Evaluation of stability under UV conditions

To evaluate the chemical/physical stability of all formulations upon UV exposure, each formulation was exposed to a total of 8 Wh/m² of UVA light in a 25 °C photostability chamber. The same set of formulations, except for the presence of UV exposure, were prepared as a non-UV exposure control (UV-control) and incubated at 25° C for the same amount of time without UV light exposure. Then, the properties and presence of visible particles of all formulations were evaluated. As a result, it was confirmed that there was no change in the properties of all formulations of Example 1 due to UV exposure.

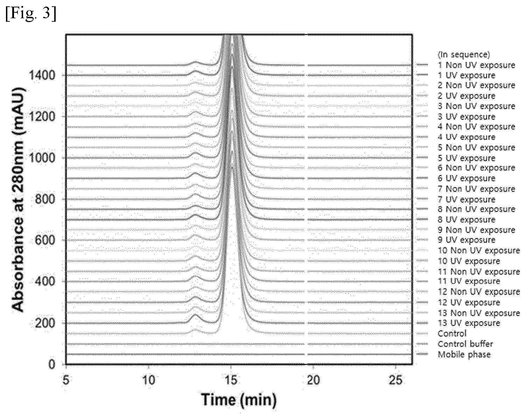

In addition, SE-HPLC analysis (Hewlett Packard Agilent 1100 Series, column; TSK-GEL G3000SWXL, 7.8 X 30 (ID mm X L cm) (Tosoh, Cat. # 08541), mobile phase: 200 mM KPi, 250 mM KCl, pH 6.2, Flow rate: 0.5 mL/min, wavelength: 280 nm) was used to observe physical degradation of each formulation before and after UV exposure. To monitor the accuracy of the assay and make a comparison between formulations, the chromatographic profile of each formulation was checked along with the injection of the control. The stability of each formulation was compared and analyzed by confirming the formation of aggregates and fragments by peak percentage according to UV exposure. As a result, it was confirmed that all formulations of Example 1 showed an insignificant increase in related substances after exposure to ultraviolet rays and, thus, were stable, as shown in FIG. 3.

In addition, cIEF analysis (Maurice cIEF; Maurice-OBM, 2AHGG-Maurice, Protein Sample, 1.0 min at 1500 V, 6 min at 3000 V; exposure: 0.005 sec at A280 nm; sample load: 55 sec; cartridge Temperature: room temperature; sample temperature: 10 °C) was performed for each formulation to monitor charge-based chemical transformation during UV exposure.

To monitor the accuracy of the assay and provide a comparison between formulations, formulations were analyzed along with the injection of the control, and the profile of each formulation was identified. The stability of each formulation was compared and analyzed by confirming changes in isomers by peak percentage according to UV exposure. As a result, no particular change in isomers was observed in all formulations of Example 1, as shown in Table 4. As such, it was confirmed that the ophthalmic liquid composition of Example 1 was stable against UV exposure.

**[Table 4]**

| **Formulation** | **Conditions for treatment** | **Area (%)** | | | | | | | | | |
|---|---|---|---|---|---|---|---|---|---|---|---|
| | | **Peak 1** | **Peak 2** | **Peak 3** | **Peak 4** | **Peak 5** | **Peak 6** | **Peak 7** | **Peak 8** | **Peak 9** | **Peak 10** |
| Control (-70 °C) | | 16.8 | 6.9 | 15.5 | 16.7 | 12.1 | 6.4 | 7.9 | 11.1 | 5.7 | 1.0 |
| 1 | Non-UV exposure | 16.6 | 8.9 | 11.4 | 20.2 | 10.5 | 6.2 | 8.1 | 10.9 | 6.0 | 1.2 |
| | UV exposure | 16.5 | 6.8 | 16.2 | 19.4 | 10.9 | 6.2 | 7.9 | 10.6 | 5.1 | 0.3 |
| 2 | Non-UV exposure | 16.7 | 9.3 | 11.8 | 17.2 | 12.2 | 6.2 | 8.8 | 11.0 | 5.6 | 1.1 |
| | UV exposure | 16.8 | 6.7 | 14.6 | 17.3 | 13.0 | 7.0 | 7.2 | 10.8 | 5.8 | 0.9 |
| 3 | Non-UV exposure | 17.8 | 7.9 | 13.1 | 16.0 | 13.1 | 5.1 | 9.0 | 11.3 | 5.6 | 1.1 |
| | UV exposure | 16.8 | 7.3 | 16.1 | 15.7 | 11.1 | 6.6 | 7.7 | 11.5 | 6.0 | 1.2 |
| 4 | Non-UV exposure | 16.6 | 8.2 | 12.4 | 20.1 | 10.4 | 6.3 | 8.1 | 11.1 | 5.6 | 1.2 |
| | UV exposure | 16.9 | 6.9 | 15.2 | 16.7 | 12.1 | 6.0 | 8.6 | 11.0 | 5.6 | 0.9 |
| 5 | Non-UV exposure | 16.5 | 11.1 | 10.8 | 18.5 | 10.8 | 6.4 | 8.3 | 11.2 | 5.3 | 0.9 |
| | UV exposure | 16.9 | 8.5 | 16.1 | 14.9 | 12.0 | 6.6 | 7.8 | 10.8 | 5.4 | 1.1 |
| 6 | Non-UV exposure | 17.1 | 7.9 | 14.3 | 16.3 | 12.7 | 6.3 | 7.7 | 11.2 | 5.5 | 1.0 |
| | UV exposure | 16.4 | 9.5 | 11.0 | 18.5 | 12.6 | 6.3 | 8.0 | 10.8 | 5.8 | 1.1 |
| 7 | Non-UV exposure | 16.9 | 7.7 | 13.3 | 19.1 | 11.1 | 6.7 | 7.0 | 11.5 | 5.5 | 1.4 |
| | UV exposure | 17.7 | 6.9 | 15.5 | 16.8 | 12.0 | 7.6 | 6.3 | 10.6 | 5.4 | 1.1 |
| 8 | Non-UV exposure | 16.1 | 7.9 | 15.6 | 16.8 | 12.0 | 5.9 | 8.8 | 11.2 | 5.1 | 0.8 |
| | UV exposure | 16.9 | 7.3 | 14.7 | 17.2 | 12.9 | 7.3 | 7.1 | 10.6 | 5.3 | 0.7 |
| 9 | Non-UV exposure | 16.5 | 8.2 | 13.6 | 19.4 | 10.3 | 6.2 | 8.2 | 11.3 | 5.5 | 1.0 |
| | UV exposure | 17.2 | 7.8 | 13.8 | 18.0 | 12.6 | 6.5 | 7.1 | 10.7 | 5.4 | 1.0 |
| 10 | Non-UV exposure | 16.3 | 9.5 | 12.3 | 20.2 | 9.9 | 6.0 | 8.3 | 11.2 | 5.5 | 1.0 |
| | UV exposure | 17.2 | 6.7 | 16.0 | 19.4 | 10.1 | 6.7 | 7.2 | 10.8 | 5.1 | 0.8 |
| 11 | Non-UV exposure | 16.7 | 8.5 | 14.0 | 19.5 | 10.4 | 7.0 | 7.1 | 11.3 | 5.1 | 0.5 |
| | UV exposure | 17.2 | 7.7 | 15.3 | 16.9 | 12.5 | 6.6 | 7.1 | 10.8 | 5.1 | 0.8 |
| 12 | Non-UV exposure | 16.5 | 9.0 | 12.6 | 19.3 | 10.8 | 6.3 | 7.7 | 11.6 | 5.3 | 1.0 |
| | UV exposure | 17.3 | 9.1 | 13.3 | 18.2 | 12.4 | 5.4 | 8.3 | 10.4 | 5.0 | 0.6 |
| 13 | Non-UV exposure | 17.1 | 7.2 | 14.2 | 18.9 | 10.7 | 6.3 | 8.0 | 10.7 | 5.8 | 1.1 |
| | UV exposure | 16.8 | 6.1 | 17.2 | 18.0 | 10.0 | 6.4 | 8.6 | 10.5 | 5.6 | 0.8 |

### Example 4: Evaluation of stability according to storage temperature conditions

The stability evaluation analysis was performed on each formulation at a certain time point after all formulations were incubated at temperatures of -70 °C, 5 °C, 25 °C and 40 °C, respectively.

### 4-1. visual inspection

At a specific time point, samples of all formulations were taken out from the storage location at each temperature, and stability was evaluated by visually checking the properties and presence of visible particles. Visual inspection was performed for each formulation under a black and white background and a white light source (13 W fluorescent tube), and digital photographs of all formulations were taken at each time point.

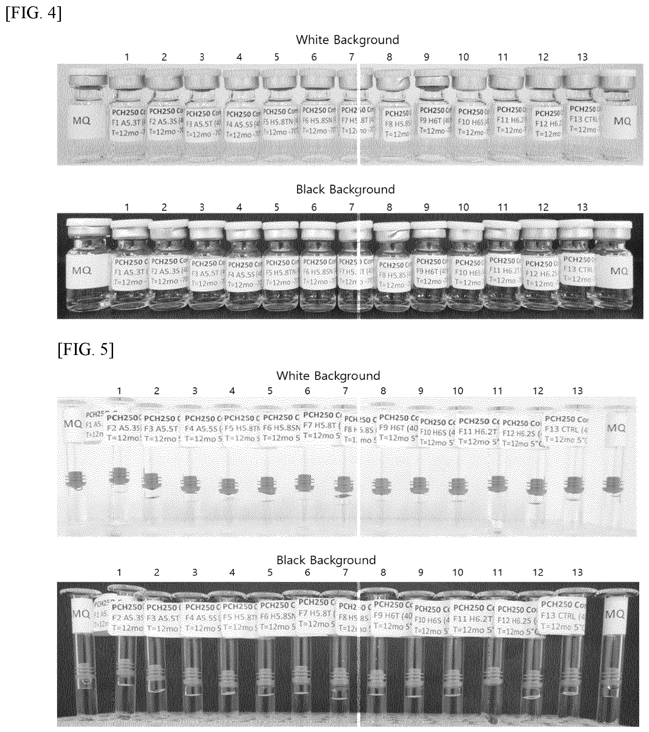

As a result, all formulations of Example 1 showed no change in properties until 12 months under freezing and refrigerating temperature conditions of -70 ° C and 5 ° C, respectively, for 6 months at 25 ° C (accelerated condition), and for 8 weeks at 40 °C (stressed conditions), as shown in FIGS. 4 and 5.

### 4-2. Measurement of concentration

The concentrations of the formulations stored at each temperature were analyzed, and the trend of concentration change and stability of the formulations for 8 weeks were checked. The concentration of aflibercept was measured by measuring the absorbance at 280 nm by using Implen Nanophotometer N50 Touch (A280) and dividing the measured values by the extinction coefficient (1.15 M⁻¹ cm⁻¹).

As a result, all formulations of Example 1 were stable in concentration for up to 12 months under each temperature condition of -70 °C and 5 °C, and for 6 months at 25 °C (accelerated condition), and the protein concentration was stable for 8 weeks at 40 °C (stressed condition), as shown in Table 5.

**[Table 5]**

| **Formulation** | **Concentration of protein by storage conditions (mg/mL)** | | | | |
|---|---|---|---|---|---|
| | **0 month** | **-70 °C, 12 months** | **5 °C, 12 months** | **25 °C, 6 months** | **40 °C, 8 weeks** |
| 1 | 40.4 | 39.7 | 39.3 | 40.4 | 40.2 |
| 2 | 40.2 | 41.9 | 39.0 | 40.4 | 39.9 |
| 3 | 39.2 | 39.2 | 38.7 | 40.1 | 38.4 |
| 4 | 39.3 | 40.2 | 39.3 | 39.8 | 38.8 |
| 5 | 39.3 | 39.7 | 38.1 | 38.9 | 38.8 |
| 6 | 40.2 | 41.6 | 40.1 | 39.4 | 39.8 |
| 7 | 40.3 | 40.2 | 39.8 | 39.8 | 40.0 |
| 8 | 39.1 | 39.7 | 40.2 | 39.3 | 38.8 |
| 9 | 39.4 | 41.6 | 41.6 | 39.5 | 39.2 |
| 10 | 38.9 | 39.0 | 41.3 | 38.9 | 38.5 |
| 11 | 39.8 | 36.5 | 39.3 | 38.6 | 39.0 |
| 12 | 39.3 | 40.1 | 39.9 | 39.6 | 39.4 |
| 13 | 40.0 | 40.4 | 40.1 | 40.1 | 40.4 |

### 4-3. Osmolality

Osmolality was measured after the preparation of all formulations. Osmolality was measured only at the time point of T=0 by using Model 2020 Freezing Point Osmometer (Advanced Instruments). As a result, most formulations were measured to have an osmolality of 345 to 382 mOsm/kg, and it was confirmed that there was no problem of using them as ophthalmic compositions.

### 4-4. pH

Stability was evaluated by measuring the pH of the formulations stored at each temperature and comparing them with the control formulations. The pH was measured by using SympHony^{®} pH Meter (VWR Scientific, catalog # SB70P). The pH was measured at room temperature, without adjusting the temperature of each formulation, and the pH meter was used after confirming that the calibration slope was 95% or higher.

As a result, all formulations of Example 1 showed a stable pH for up to 12 months under each temperature condition of -70 °C and 5 °C, and for 6 months at 25 °C (accelerated condition), and the pH was stable for 8 weeks at 40 °C (stressed conditions), as shown in Table 6.

**[Table 6]**

| **Formulation** | **pH by storage conditions** | | | | |
|---|---|---|---|---|---|
| | **0 month** | **-70 °C, 12 months** | **5 °C, 12 months** | **25 °C, 6 months** | **40 °C, 8 weeks** |
| 1 | 5.34 | 5.32 | 5.32 | 5.25 | 5.33 |
| 2 | 5.38 | 5.36 | 5.34 | 5.25 | 5.31 |
| 3 | 5.45 | 5.49 | 5.45 | 5.41 | 5.46 |
| 4 | 5.46 | 5.45 | 5.44 | 5.39 | 5.55 |
| 5 | 5.74 | 5.76 | 5.77 | 5.71 | 5.81 |
| 6 | 5.75 | 5.78 | 5.78 | 5.70 | 5.83 |
| 7 | 5.79 | 5.81 | 5.82 | 5.74 | 5.82 |
| 8 | 5.81 | 5.83 | 5.85 | 5.72 | 5.86 |
| 9 | 5.96 | 6.00 | 6.00 | 5.97 | 5.99 |
| 10 | 6.04 | 6.02 | 6.01 | 5.94 | 5.98 |
| 11 | 6.13 | 6.18 | 6.18 | 6.13 | 6.15 |
| 12 | 6.14 | 6.20 | 6.20 | 6.12 | 6.16 |
| 13 | 6.08 | 6.14 | 6.16 | 6.09 | 6.08 |

### 4-5. SE-HPLC analysis

Physical degradation of each formulation was observed during storage at a particular temperature through SE-HPLC analysis (Hewlett Packard Agilent 1100 Series, column; TSK-GEL G3000SWXL, 7.8 X 30 (ID mm X L cm) (Tosoh, Cat. # 08541), mobile phase: 200 mM KPi, 250 mM KCl, pH 6.2, flow rate: 0.5 mL/min, wavelength: 280 nm). To monitor the accuracy of the assay and provide a comparison between formulations, the chromatographic profile of each formulation was checked along with the injection of the control. The stability of each formulation was compared and analyzed by confirming the formation of aggregates and fragments by peak percentage over time at each temperature.

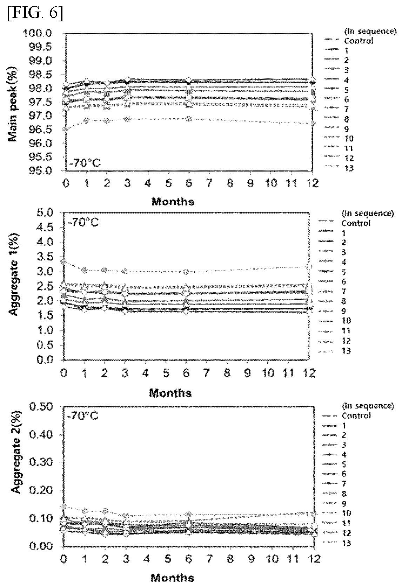

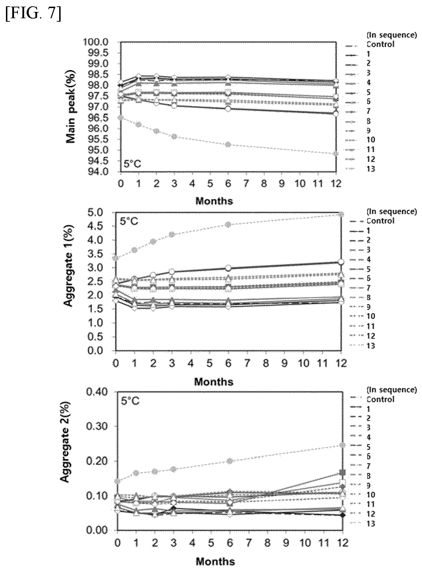

As a result, all formulations of Example 1 did not show an increase or decrease in aggregates or fragments for 12 months at -70 °C (frozen conditions), while maintaining the status, as shown in FIG. 6. Formulations 5 and 6 of Example 1 to which NaCl was added for 12 months showed the highest increase in aggregates at 5 °C (refrigerated condition), as compared to other formulations, as shown in FIG. 7. That is, it was confirmed that the composition free of a salt (NaCl) was more stable.

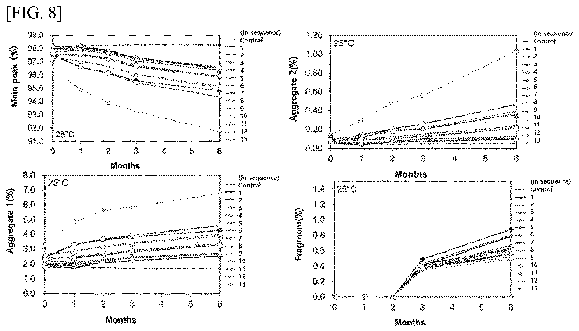

Formulations 5 and 6 to which NaCl was added showed the highest increase rate of aggregates for 6 months at 25 °C (accelerated condition), as in the refrigerated condition, as shown in FIG. 8 and Table 7. In addition, it was confirmed that formulations with a lower pH (Formulations 1 and 2) showed a greater increase rate of fragments after 3 months, and formulations with a higher pH (Formulations 11 and 12) showed a greater increase rate of aggregates.

**[Table 7]**

| **Formulation** | **Conditions** | **Changes in aggregates and fragments under accelerated conditions (25 °C, 6 months)** | | | |
|---|---|---|---|---|---|
| | | **Aggregate 2 (%)** | **Aggregate 1 (%)** | **Main Peak (%)** | **Fragments (%)** |
| 1 | 0 month | 0.1 | 1.9 | 98.0 | - |
| | 25 °C, 6 months | 0.1 | 2.5 | 96.5 | 0.9 |
| 2 | 0 month | 0.1 | 1.8 | 98.1 | - |
| | 25 °C, 6 months | 0.1 | 2.5 | 96.6 | 0.8 |
| 3 | 0 month | 0.1 | 2.2 | 97.7 | - |
| | 25 °C, 6 months | 0.1 | 2.7 | 96.4 | 0.8 |
| 4 | 0 month | 0.1 | 2.1 | 97.9 | - |
| | 25 °C, 6 months | 0.1 | 2.7 | 96.6 | 0.7 |
| 5 | 0 month | 0.1 | 2.4 | 97.5 | - |
| | 25 °C, 6 months | 0.4 | 4.2 | 94.8 | 0.6 |
| 6 | 0 month | 0.1 | 2.4 | 97.5 | - |
| | 25 °C, 6 months | 0.5 | 4.6 | 94.3 | 0.6 |
| 7 | 0 month | 0.1 | 2.4 | 97.5 | - |
| | 25 °C, 6 months | 0.2 | 3.3 | 95.9 | 0.6 |
| 8 | 0 month | 0.1 | 2.4 | 97.6 | - |
| | 25 °C, 6 months | 0.2 | 3.3 | 95.9 | 0.6 |
| 9 | 0 month | 0.1 | 2.4 | 97.6 | - |
| | 25 °C, 6 months | 0.2 | 3.3 | 95.9 | 0.6 |
| 10 | 0 month | 0.1 | 2.3 | 97.6 | - |
| | 25 °C, 6 months | 0.2 | 3.3 | 96.0 | 0.6 |
| 11 | 0 month | 0.1 | 2.6 | 97.3 | - |
| | 25 °C, 6 months | 0.4 | 3.9 | 95.2 | 0.6 |
| 12 | 0 month | 0.1 | 2.6 | 97.3 | - |
| | 25 °C, 6 months | 0.4 | 4.0 | 95.1 | 0.5 |
| 13 | 0 month | 0.1 | 3.3 | 96.5 | - |
| | 25 °C, 6 months | 1.0 | 6.8 | 91.7 | 0.5 |

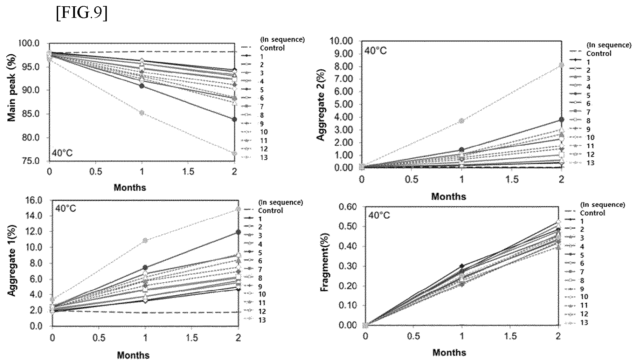

The formulations showed a faster decomposition rate at 40 °C (stressed conditions) than at 25 °C but showed a generally similar trend, as shown in FIG. 9 and Table 8.

**[Table 8]**

| **Formulation** | **Conditions** | **Changes in aggregates and fragments under stressed conditions (40 °C, 8 weeks)** | | | |
|---|---|---|---|---|---|
| | | **Aggregate 2 (%)** | **Aggregate 1 (%)** | **Main Peak (%)** | **Fragments (%)** |
| 1 | 0 month | 0.1 | 1.9 | 98.0 | - |
| | 40 °C, 8 weeks | 0.4 | 4.7 | 94.4 | 0.5 |
| 2 | 0 month | 0.1 | 1.8 | 98.1 | - |
| | 40 °C, 8 weeks | 0.4 | 5.0 | 94.0 | 0.5 |
| 3 | 0 month | 0.1 | 2.2 | 97.7 | - |
| | 40 °C, 8 weeks | 0.6 | 5.6 | 93.3 | 0.5 |
| 4 | 0 month | 0.1 | 2.1 | 97.9 | - |
| | 40 °C, 8 weeks | 0.6 | 5.8 | 93.0 | 0.5 |
| 5 | 0 month | 0.1 | 2.4 | 97.5 | - |
| | 40 °C, 8 weeks | 3.8 | 11.9 | 83.8 | 0.5 |
| 6 | 0 month | 0.1 | 2.4 | 97.5 | - |
| | 40 °C, 8 weeks | 2.3 | 9.0 | 88.3 | 0.4 |
| 7 | 0 month | 0.1 | 2.4 | 97.5 | - |
| | 40 °C, 8 weeks | 1.0 | 6.2 | 92.4 | 0.4 |
| 8 | 0 month | 0.1 | 2.4 | 97.6 | - |
| | 40 °C, 8 weeks | 1.0 | 6.3 | 92.2 | 0.4 |
| 9 | 0 month | 0.1 | 2.4 | 97.6 | - |
| | 40 °C, 8 weeks | 1.5 | 7.0 | 91.1 | 0.4 |
| 10 | 0 month | 0.1 | 2.3 | 97.6 | - |
| | 40 °C, 8 weeks | 1.8 | 7.5 | 90.3 | 0.5 |
| 11 | 0 month | 0.1 | 2.6 | 97.3 | - |
| | 40 °C, 8 weeks | 2.7 | 8.4 | 88.5 | 0.4 |
| 12 | 0 month | 0.1 | 2.6 | 97.3 | - |
| | 40 °C, 8 weeks | 3.1 | 9.1 | 87.4 | 0.5 |
| 13 | 0 month | 0.1 | 3.3 | 96.5 | - |
| | 40 °C, 8 weeks | 8.1 | 14.9 | 76.6 | 0.4 |

Through the above-described analysis results, it was confirmed that the formation rate of aggregates was high when the pH was 6.2 or more, and the formation rate of fragments increased when the pH was 5.3 or less, and, therefore, the formulation free of a salt (NaCl) was remarkably stable.

### 4-6. cIEF analysis

For each formulation, cIEF analysis was performed by using Protein Simple Maurice (Maurice cIEF; Maurice-OBM, 2AHGG-Maurice, Protein Sample, 1.0 min at 1500 V, 6 min at 3000 V; exposure: 0.005 sec at A280 nm; sample load: 55 sec; cartridge temperature: room temperature; sample temperature: 10 °C) to monitor charge-based chemical transformation during storage at a specific temperature. To monitor the accuracy of the assay and provide a comparison between formulations, formulations were analyzed along with the injection of the controls, and the profile of each formulation was identified. The stability of each formulation was compared and analyzed by confirming the change in isomers by peak percentage over time at each temperature.

As a result, the relative acidic peak (peaks 1 to 4) in most formulations increased at 40 °C (stressed conditions), and the relative neutral peak (peaks 5 to 6) and relative basic peak (peak 7 to 10) showed a decreasing trend, as shown in Table 9. In addition, formulations with a high pH (Formulations 9 to 12 with a pH of 6.0 to 6.2) showed a greater increase in acidic peak at a high temperature of 40 °C, as compared to formulations with a lower pH, and formulations containing NaCl (Formulations 5 and 6) were found to have a higher relative acidic peak ratio than those of the formulations free of NaCl.

Through these results, it was confirmed that the formulations with a pH less than 6.0 and free of a salt were more stable than formulations under other conditions. Considering such results in combination with the SE-HPLC analysis results of Examples 4-5, pH 5.5 to pH 5.8, it was confirmed that the formulations with a pH of 5.5 to 5.8 and free of a salt had remarkably excellent stability.

**[Table 9]**

| **Formulatio n** | **Condition s for treatment** | **Changes in isomers under stressed conditions (40 °C, 8 weeks), Area (%)** | | | | | | | | | |
|---|---|---|---|---|---|---|---|---|---|---|---|
| | | **Pea k 1** | **Pea k2** | **Pea k3** | **Pea k 4** | **Pea k 5** | **Pea k6** | **Pea k7** | **Pea k 8** | **Pea k9** | **Pea k 10** |
| 1 | 0 month | 16.6 | 6.5 | 15.5 | 17.2 | 13.5 | 8.1 | 5.7 | 11.0 | 5.4 | 0.6 |
| | 40 °C, 8 weeks | 17.3 | 7.4 | 20.0 | 15.4 | 10.6 | 5.7 | 7.9 | 9.4 | 5.5 | 0.9 |
| 2 | 0 month | 16.2 | 7.2 | 16.7 | 14.8 | 13.4 | 7.2 | 6.8 | 10.8 | 5.8 | 1.1 |
| | 40 °C, 8 weeks | 16.8 | 10.5 | 18.7 | 150 | 10.1 | 6.5 | 7.3 | 9.0 | 5.2 | 0.8 |
| 3 | 0 month | 15.6 | 6.4 | 13.5 | 19.0 | 13.6 | 7.9 | 5.9 | 11.1 | 5.9 | 1.0 |
| | 40 °C, 8 weeks | 18.6 | 10.2 | 17.3 | 14.9 | 12.0 | 6.1 | 6.5 | 9.0 | 4.7 | 0.7 |
| 4 | 0 month | 16.9 | 6.3 | 16.4 | 16.6 | 13.2 | 6.7 | 7.0 | 10.7 | 5.4 | 0.8 |
| | 40 °C, 8 weeks | 18.1 | 11.2 | 19.0 | 14.7 | 9.9 | 6.3 | 6.6 | 8.7 | 4.8 | 0.8 |
| 5 | 0 month | 16.7 | 6.3 | 14.3 | 18.5 | 12.9 | 7.4 | 6.4 | 11.2 | 5.2 | 1.1 |
| | 40 °C, 8 weeks | 19.3 | 13.2 | 16.7 | 16.1 | 9.8 | 5.7 | 6.6 | 7.7 | 4.3 | 0.8 |
| 6 | 0 month | 16.1 | 6.8 | 14.2 | 18.1 | 12.6 | 7.3 | 6.9 | 11.0 | 5.6 | 1.4 |
| | 40 °C, 8 weeks | 18.6 | 8.5 | 22.2 | 14.0 | 10.1 | 7.2 | 5.3 | 8.6 | 4.6 | 0.9 |
| 7 | 0 month | 16.5 | 8.2 | 14.0 | 18.3 | 12.8 | 6.8 | 7.2 | 10.8 | 4.8 | 0.4 |
| | 40 °C, 8 weeks | 19.5 | 9.1 | 19.4 | 16.0 | 9.8 | 6.8 | 5.7 | 8.4 | 4.3 | 1.0 |
| 8 | 0 month | 16.0 | 7.5 | 17.1 | 18.6 | 10.5 | 7.3 | 6.9 | 106 | 5.1 | 0.5 |
| | 40 °C, 8 weeks | 20.5 | 8.6 | 21.5 | 12.5 | 11.3 | 6.6 | 6.2 | 8.0 | 4.2 | 0.7 |
| 9 | 0 month | 16.9 | 7.1 | 17.2 | 16.2 | 12.9 | 7.3 | 6.9 | 10.7 | 4.4 | 0.2 |
| | 40 °C, 8 weeks | 20.9 | 9.8 | 21.6 | 12.5 | 10.9 | 6.6 | 5.6 | 7.7 | 3.8 | 0.7 |
| 10 | 0 month | 17.6 | 6.3 | 15.7 | 20.5 | 10.7 | 6.2 | 7.7 | 10.6 | 4.5 | 0.2 |
| | 40 °C, 8 weeks | 21.9 | 12.6 | 18.4 | 14.9 | 9.4 | 6.0 | 5.4 | 7.1 | 3.7 | 0.7 |
| 11 | 0 month | 16.5 | 6.3 | 14.6 | 18.3 | 12.4 | 7.8 | 6.3 | 11.3 | 5.3 | 1.1 |
| | 40 °C, 8 weeks | 23.4 | 13.3 | 21.0 | 12.6 | 8.9 | 5.6 | 4.6 | 6.9 | 3.1 | 0.6 |
| 12 | 0 month | 17.4 | 5.6 | 15.1 | 18.0 | 13.0 | 6.6 | 7.4 | 10.6 | 5.5 | 1.0 |
| | 40 °C, 8 weeks | 22.9 | 16.0 | 18.7 | 12.2 | 9.9 | 5.6 | 4.7 | 6.3 | 3.1 | 0.7 |
| 13 | 0 month | 16.5 | 6.4 | 15.2 | 17.5 | 13.3 | 7.4 | 6.8 | 10.5 | 5.5 | 0.9 |
| | 40 °C, 8 weeks | 23.2 | 13.8 | 17.3 | 17.7 | 8.8 | 5.2 | 4.4 | 5.5 | 3.5 | 0.7 |

### 4-7. FlowCAM analysis

FlowCAM analysis was performed by using a FlowCAM particle imaging system (Benchtop B3 Series, Fluid Imaging Technologies) to monitor the level of subvisible particles in each formulation during storage at a certain temperature. The stability of each formulation was evaluated by checking changes in fine particles over time at each temperature. As a result, a low number of subvisible particles were detected in all formulations of Example 1, and no increase or decrease in the number of subvisible particles over time at each temperature was observed.

### 4-8. RP-HPLC analysis

Among the formulations in Example 1, Formulations 2, 4, 8 and 13 were stored for 6 months at 5 °C (refrigerated, long-term storage conditions), 25 °C (accelerated conditions) and 40 °C (severe conditions), and it was checked whether or not an oxidized form that may affect their activities (e.g., potencies) is generated and changes by using RP-HPLC analysis at each time point (Waters, Acquity UPLC H-class system, column; ACQUITY UPLC Protein BEH C4 Column, 2.1 X 100 (ID mm X L cm) (Waters, Cat. # 186004496), mobile phase A: 0.1% TFA, mobile phase B: 0.08% TFA in ACN, flow rate: 0.5 mL/min, wavelength: 214 nm). All formulations showed generation of almost no oxidant for 6 months at 5 °C and generation of some oxidants at 25 °C such that the lower the pH is, the smaller the difference in the degree of generation of oxidants the formulations showed, and the formulations with a low pH showed a significantly increase in the generation of oxidants at 40 °C, as shown in Table 10.

**[Table 10]**

| **Formulation** | **0 month** | | **Storage conditions (6 months, 5 °C)** | | **Accelerated conditions (6 months, 25 °C)** | | **Stressed conditions (6 months, 40 °C)** | |
|---|---|---|---|---|---|---|---|---|
| | **Main peak (%)** | **Oxidants (%)** | **Main peak (%)** | **Oxidants (%)** | **Main peak (%)** | **Oxidants (%)** | **Main peak (%)** | **Oxidants (%)** |
| 2 | 100.00 | 0 | 99.98 | 0.02 | 99.94 | 0.06 | 98.90 | 1.10 |
| 4 | 100.00 | 0 | 99.98 | 002 | 99.96 | 0.04 | 99.17 | 0.83 |
| 8 | 100.00 | 0 | 99.98 | 0.02 | 99.98 | 0.02 | 99.34 | 0.66 |
| 13 | 100.00 | 0 | 99.92 | 0.08 | 99.97 | 0.03 | 99.60 | 0.40 |

### 4-9. Deamidation assay

Among the formulations of Example 1, Formulations 2, 4, 8 and 13 were stored for 6 months at 5 °C (refrigerated, long-term storage conditions), 25 °C (accelerated conditions), 40 °C (stressed conditions), and deamidation analysis was performed to evaluate whether or not proteins were degraded. ISOQUANT^{®} Isoaspartate Detection Kit (Promega, MA1010) was used for pretreatment during analysis, and, after preparation of samples, HPLC analysis (Waters, HPLC e2695, column; Synergi^{™} Hydro-RP, 4.6 x 150 (ID mm X L cm) (Phenomenex, Cat. # 00F-4375-E0), mobile phase: 50 mM KPi, pH 6.20, flow rate: 1.0 ml/min, wavelength: 260 nm) was performed. It was confirmed that all formulations showed similar results at 5 °C, and a similar increase rate at 25 °C, and at 40 °C, and Formulations 2, 4 and 8 showed a lower increase rate than that of Formulation 13, as shown in Table 11.

**[Table 11]**

| **Formulation** | **Deamidation (mol/mol of Aflibercept)** | | | |
|---|---|---|---|---|
| | **0 month** | **Storage conditions (6 months, 5 °C)** | **Accelerated conditions (6 months, 25 °C)** | **Stressed conditions (6 months, 40 °C)** |
| 2 | 0.32 | 0.32 | 0.79 | 3.49 |
| 4 | 0.31 | 0.32 | 0.70 | 3.44 |
| 8 | 0.30 | 0.41 | 0.71 | 3.47 |
| 13 | 0.33 | 0.43 | 0.91 | 4.12 |

### 4-10. Binding assay

Among the formulations of Example 1, Formulations 2, 4, 8 and 13 were stored for 6 months at 5 °C (refrigerated, long-term storage conditions), 25 °C (accelerated conditions), 40 °C (severe conditions), and the degree of binding of aflibercept to the target protein VEGF was confirmed as activities through the binding assay (ELISA method, coating protein: VEGF165 protein, aflibercept concentration range: 0.04 to 500 ng/mL, standard product: control drug (EYLEA)). As the activities decrease, the percentage of EC₅₀ increases, as compared to the standard product. As a result, it was confirmed the activities of all formulations was measured in the range of 80 to 125% at 5 °C, and the percentage of EC₅₀ increased in all formulations at 25 °C and 40 °C, and the lower the pH is, the more the activities decreased, as shown in Table 12.

**[Table 12]**

| **Formulation** | **Percentage of EC₅₀ as compared to standard product** | | | |
|---|---|---|---|---|
| | **0 month** | **Storage conditions (6 months, 5 °C)** | **Accelerated conditions (6 months, 25 °C)** | **Stressed conditions (6 months, 40 °C)** |
| 2 | 101 | 91 | 167 | 181 |
| 4 | 106 | 102 | 111 | 152 |
| 8 | 115 | 98 | 96 | 137 |
| 13 | 102 | 98 | 106 | 138 |

From the above description, those skilled in the art to which the present invention belongs to can understand that the present invention may be embodied in other specific forms, without changing its technical idea or essential features. In this regard, it should be understood that the embodiments described above are illustrative in all respects, and the present invention is not limited thereto. The scope of the present invention should be construed as comprising all changes or modifications derived from the meaning and scope of the claims to be described below and equivalent concepts rather than the detailed description above.

## Claims

1. An ophthalmic liquid composition comprising a therapeutically effective amount of aflibercept; a buffer containing histidine; a sugar including sucrose, trehalose or a combination thereof; and a surfactant,
wherein the surfactant is 0.01% (w/v) of polysorbate or poloxamer,
wherein the composition has a pH 5.8, and
wherein the composition contains no sodium chloride.

2. The ophthalmic liquid composition of claim 1, wherein the sodium acetate or histidine is present at a concentration of 5 to 20 mM.

3. The ophthalmic liquid composition of claim 1, wherein the sugar is present at a concentration of 5 to 20% (w/v).

4. The ophthalmic liquid composition of claim 1, wherein the composition is suitable for intravitreal injection.

5. A composition for stabilizing aflibercept, comprising a buffer containing histidine; a sugar including sucrose, trehalose or a combination thereof; and a surfactant,
wherein the surfactant is 0.01% of polysorbate or poloxamer,
wherein the composition has a pH 5.8, and
wherein the composition contains no sodium chloride.

6. The composition for stabilizing aflibercept of claim 5, wherein the sodium acetate or histidine at a concentration of 5 to 20 mM.

7. The composition for stabilizing aflibercept of claim 5, wherein the sugar is present at a concentration of 5 to 20% (w/v).

8. A container comprising the ophthalmic liquid composition of any one of claims 1 to 4.

9. The container of claim 8, wherein the container is a vial or a prefilled syringe.

10. The ophthalmic liquid composition of any one of claims 1 to 4 for use in preventing or treating ophthalmic diseases.

## Patentansprüche

1. Ophthalmische flüssige Zusammensetzung, umfassend eine therapeutisch wirksame Menge von Aflibercept; einen histidinhaltigen Puffer; einen Zucker, der Saccharose, Trehalose oder eine Kombination davon beinhaltet; und ein Tensid,
wobei das Tensid 0,01 % (w/v) Polysorbat oder Poloxamer ist,
wobei die Zusammensetzung einen pH-Wert von 5,8 aufweist und
wobei die Zusammensetzung kein Natriumchlorid enthält.

2. Ophthalmische flüssige Zusammensetzung nach Anspruch 1, wobei das Natriumacetat oder Histidin in einer Konzentration von 5 bis 20 mM vorliegt.

3. Ophthalmische flüssige Zusammensetzung nach Anspruch 1, wobei der Zucker in einer Konzentration von 5 bis 20 % (w/v) vorliegt.

4. Ophthalmische flüssige Zusammensetzung nach Anspruch 1, wobei die Zusammensetzung zur intravitrealen Injektion geeignet ist.

5. Zusammensetzung zum Stabilisieren von Aflibercept, umfassend einen histidinhaltigen Puffer; einen Zucker, der Saccharose, Trehalose oder eine Kombination davon beinhaltet; und ein Tensid,
wobei das Tensid 0,01 % Polysorbat oder Poloxamer ist,
wobei die Zusammensetzung einen pH-Wert von 5,8 aufweist und
wobei die Zusammensetzung kein Natriumchlorid enthält.

6. Zusammensetzung zum Stabilisieren von Aflibercept nach Anspruch 5, wobei das Natriumacetat oder Histidin in einer Konzentration von 5 bis 20 mM vorliegt.

7. Zusammensetzung zum Stabilisieren von Aflibercept nach Anspruch 5, wobei der Zucker in einer Konzentration von 5 bis 20 % (w/v) vorliegt.

8. Behälter, umfassend die ophthalmische flüssige Zusammensetzung nach einem der Ansprüche 1 bis 4.

9. Behälter nach Anspruch 8, wobei es sich bei dem Behälter um ein Fläschchen oder eine vorgefüllte Spritze handelt.

10. Ophthalmische flüssige Zusammensetzung nach einem der Ansprüche 1 bis 4 zur Verwendung beim Vorbeugen oder Behandeln von ophthalmischen Erkrankungen.

## Revendications

1. Composition liquide ophtalmique comprenant une quantité thérapeutiquement efficace d'aflibercept ; un tampon contenant de l'histidine ; un sucre comprenant du saccharose, du tréhalose ou une combinaison de ceux-ci ; et un tensioactif,
dans laquelle le tensioactif est 0,01% (p/v) de polysorbate ou de poloxamère,
dans laquelle la composition a un pH de 5,8, et
dans laquelle la composition ne contient pas de chlorure de sodium.

2. Composition liquide ophtalmique de la revendication 1, dans laquelle l'acétate de sodium ou l'histidine est présent(e) à une concentration de 5 à 20 mM.

3. Composition liquide ophtalmique de la revendication 1, dans laquelle le sucre est présent à une concentration de 5 à 20% (p/v).

4. Composition liquide ophtalmique de la revendication 1, dans laquelle la composition est appropriée pour une injection intravitréenne.

5. Composition pour stabiliser l'aflibercept, comprenant un tampon contenant de l'histidine ; un sucre comprenant du saccharose, du tréhalose ou une combinaison de ceux-ci ; et un tensioactif,
dans laquelle le tensioactif est 0,01% de polysorbate ou de poloxamère,
dans laquelle la composition a un pH de 5,8, et
dans laquelle la composition ne contient pas de chlorure de sodium.

6. Composition pour stabiliser l'aflibercept de la revendication 5, dans laquelle l'acétate de sodium ou l'histidine est à une concentration de 5 à 20 mM.

7. Composition pour stabiliser l'aflibercept de la revendication 5, dans laquelle le sucre est présent à une concentration de 5 à 20% (p/v).

8. Récipient comprenant la composition liquide ophtalmique de l'une quelconque des revendications 1 à 4.

9. Récipient de la revendication 8, dans lequel le récipient est un flacon ou une seringue préremplie.

10. Composition liquide ophtalmique de l'une quelconque des revendications 1 à 4, pour une utilisation dans la prévention ou le traitement de maladies ophtalmiques.
